# EUROPEAN PATENT APPLICATION

(11) **EP 4 439 578 A1**
(43) Date of publication of application: **02.10.2024**
(21) Application number: 24166580.1
(22) Date of filing: 26.03.2024
(51) Int. Cl.: G16H 20/70, G16H 40/63

(54) **SYSTEMS AND METHODS FOR THE TREATMENT OF ONE OR MORE INDICATIONS**

(30) Priority: 27.03.2023 US 202363492458 P
(71) Applicant: Pear Therapeutics (US), Inc., Boston, MA 02109 (US)
(72) Inventor: Thorndike, Frances, 02109 Boston (US); Kersanske, Brent, 02109 Stoneham (US)
(74) Representative: Murgitroyd & Company

(57) **Abstract**

Presented herein are systems and methods for generating user interfaces based on conditions associated with users. A computing system can provide a first user interface comprising a first plurality of user interface elements to present first digital therapeutic content to a user. The first user interface can address a plurality of conditions. The computing system can identify a condition to be addressed in the user using data associated with the user. The computing system can generate a second user interface to include (i) at least one of the first plurality of user interface element and (ii) a second plurality of user interface elements to present second digital therapeutic content to address the condition. The computing system can provide the second user interface. The provision of the first and second digital therapeutic content can improve the efficacy of the medication taken by the user to address the condition.

## Description

### CROSS REFERENCE TO RELATED APPLICATIONS

The present application claims priority to U.S. Provisional Application No. 63/492,458, titled "Systems and Methods for Treatment of One or More Indications," filed March 27, 2023, the entirety of which is incorporated herein by reference.

### TECHNICAL FIELD

The present disclosure relates to digital therapeutics and, more particularly, to systems and methods for automated generation of graphical user interfaces in the context of digital therapeutics for the treatment of symptoms associated with one or more medical conditions.

### BACKGROUND

Drug therapy has played a significant role in the treatment of various medical conditions. Traditional drug therapy involves the administration of pharmaceuticals and the like. Examples of conventional pharmaceuticals may include small-molecule drugs, which are usually derived from chemical synthesis, and biopharmaceuticals, which may include recombinant proteins, vaccines, blood products used therapeutically gene therapy, monoclonal antibodies, cell therapy, and the like. While drug therapy has proven to be an effective mechanism for treating certain medical conditions, it is not without deficiencies. For example, treatment of conditions may require individual systems and methods, where each system and method require individual maintenance.

Digital therapeutics may be provided via an application running on a computing device in furtherance of addressing the medical condition of the user. One issue may be that the application may be limited to present fixed, static set of digital therapeutic content aimed at addressing one type of medical condition. Such fixed, static user interfaces may restrict the functionality of the application to the originally pre-configured capabilities, without any dynamic adjustment to account for user engagement or a particular medical condition. This may result in reduction of the efficacy of the digital therapeutic and the utility of the application to addressing the specified medical condition.

Furthermore, the fixed, static user interfaces may lead to wasted consumption of computing resources and network bandwidth from providing ineffective content when the user has other conditions besides the condition initially set out. From the perspective of the provider of the digital therapeutic, the provider may have to spend time and replicate efforts in creating interfaces and content to include in those interfaces for each individual condition. As a consequence, additional disk space may be occupied and wasted from the storage of duplicative content and interfaces, as well as individual applications for the respective conditions. In addition, these different applications may lack interoperability with respect to the data even for the same users, resulting to creation and storage of redundant data on the databases.

### SUMMARY

To address these and other technical challenges, a computing system may initially provide indication-agnostic digital therapeutic content and then dynamically and automatically generate user interfaces to provide indication-specific digital therapeutic content to address various identified conditions in a user. There may be several advantages and benefits from providing indication-agnostic and then indication-specific digital therapeutic content. The first benefit may be the ability to re-use indication-agnostic content (e.g., account login or medication adherence) across many indications to a variety of users. Configured with user interfaces to present indication-agnostic content common across a wide range of indications and indication-specific content for particular indications upon identifying which indications the user may have, the digital therapeutic application may have greater versatility, relative to applications with fixed set of content and user interfaces.

The indication-specific digital therapeutic content may also be re-used across different indications from leveraging the same core user interfaces and digital therapeutic content, with modifications added for the particular indications. This may add to the versatility of the application across a wider set of users and indications. By expanding the potential user base of the digital therapeutic application, there may be efficiencies (e.g., in terms of computation resources and network bandwidth consumption) gained from using the same core digital therapeutic content to address different conditions in different users. For example, the same digital therapeutic content may be used to address health conditions such as fatigue and nausea that are common across multiple diseases such as cancer and pregnancy. The therapeutic content may be employed across different indications that are similar, taking advantage of the core treatment as a starting point or template. For instance, the digital therapeutic content to address major depressive disorder may be modified to treat depression in users with breast cancer.

The second benefit may be in the personalization and targeting of the digital therapeutic content for a particular user. Although the core digital therapeutic content may contain similar or identical components, adjustments may be made by the application to the delivery mechanism (e.g., timing frequency, dosage, difficulty, quantity, or quality) for a specific user or user segment. Moreover, the digital therapeutic content may be augmented with additional modules or components for a specific user, when a new state (e.g., side effect, symptom, or emotion) is detected on the part of the user associated with the condition. For example, the user may be provided initially with digital therapeutic to address itching or scratching symptoms for atopic dermatitis or psoriasis. When additional data indicates that the user is experiencing fatigue, the components or modules for addressing fatigue may be added to the digital therapeutic content containing components for atopic dermatitis or psoriasis. While there is core digital therapeutic content, additional features can be added depending on factors such as a user's side effect profile that is not addressed by the core digital therapeutic treatment. In another example, a user with breast cancer may be provided with digital therapeutic treatment for fatigue as a symptom of breast cancer, and then when the user reports insomnia (e.g., via the application), the digital therapeutic content may be augmented with digital therapeutic content for insomnia.

The third benefit may be in the receipt and administration of the digital therapeutics to the involved entities. On one side, the user may be able to receive targeted, personalized treatment and the ability to streamline the treatment plan. On the other, the provider may have the ability to provide more comprehensive treatment with the reuse of digital therapeutic content across different conditions, thereby expanding available options for the digital therapeutic. The menu of options may add more candidate modules or components to be used to address various conditions on a wider segment of users. This may also allow the provider to better provide the digital therapeutic across a wider set of users, while simultaneously providing a more personalized and targeted treatment for individual users.

Furthermore, since the digital therapeutic content can be reused, the provider and user may interface with a single application. For the user, it may be easier to receive all their treatment from a single app, thereby increasing engagement and adherence. For the provider, the administrative burden may be reduced, as the provider does not have to manage each application for each individual condition. The provider may be able to rely on the application to manage interfacing with the user in order to send the user feedback to recommend an augmented digital therapeutic content and to address the new condition that has appeared. In addition, because the provider for the digital therapeutic has access to the user's profile (e.g., including any adverse effects experienced by the user), the best options for augmenting the existing digital therapeutic with additional digital therapeutic content may be selected, when a new condition is detected. The augmented digital therapeutic content may best target with more specific, personalized treatment.

To that end, the computer system may initially provide a set of user interfaces that present digital therapeutic content that is agnostic to any particular indication. By providing an indication-agnostic digital therapeutic, the computing system may use the same user interfaces (e.g., account login or medication adherence) over several, different indications to many groups of users, thereby reducing the amount of disk space and memory for storage of such interfaces. Moreover, the computing system may expand the potential user base for the application, thereby increasing the efficiencies in using the same core digital therapeutic content to address different conditions in different users.

Upon provision, the computing system may receive and collect data associated with the user, such as interaction data with the initial set of user interfaces for the indication-agnostic digital therapeutic content. As additional data associated with a user is gathered, the computing system may identify or determine the condition to be addressed in the user. Based on the identification of the condition, the computing system may dynamically or automatically generate user interfaces, for example, by adding new components or functionality to address the condition specific to the user. Since the computing system has access to the data associated with the users in interacting with the user interfaces, the computing system may automatically better select augmentations to the user interface with additional digital therapeutic content when a new condition is detected, with more specific, targeted content to address the condition in the user. The digital therapeutic content provided through the user interface can be targeted for the specific user population, including adjusting the delivery mechanism, the timing or frequency, the dosage or quantity, among others.

Furthermore, the user interfaces may be augmented to provide additional digital therapeutic content to target the indication, when the indication or symptom is detected as apparent in the user. To the core digital therapeutic content, the computing system can add additional features depending on numerous factors such as a user's side effect profile. This may allow the user to receive the targeted, personalized therapy through a single application, thereby increasing adherence and improving the quality of human-computer interactions (HCI) between the user and the application. In addition, the computational and storage burden from having separate user interfaces and different applications for each individual indication may be reduced, thereby freeing up computing resources and network bandwidth. The computing system may provide a whole host of functionalities and interactivity to provide digital therapeutics for a variety of conditions within a single application, thereby increasing the utility of the application over a wider group of different users.

Aspects of the present disclosure relate to systems and methods of generating user interfaces based on conditions of users. One or more processors can provide a first user interface comprising a first plurality of user interface elements to present first digital therapeutic content to a user. The first user interface can address one or more of a plurality of conditions via interactions with the first plurality of user interface elements. The one or more processors can identify a condition to be addressed in the user from the plurality of conditions, using data associated with the user. The one or more processors can generate a second user interface to include (i) at least one of the first plurality of user interface elements of the first user interface and (ii) a second plurality of user interface elements to present second digital therapeutic content to address the condition identified for the user. The one or more processors can provide the second user interface comprising the second plurality of user interface elements to present the second digital therapeutic content to the user to address the condition identified for the user.

In some embodiments, the one or more processors can receive input data identifying the interactions by the user with the second plurality of user interface elements of the second user interface. In some embodiments, the one or more processors can identify a second condition different from the first condition to be addressed in the user based on the input data. In some embodiments, the one or more processors can generate a third user interface to include (i) the second user interface and (ii) a third plurality of user interface elements to provide third digital therapeutic content to address the second condition identified for the user.

In some embodiments, the one or more processors can identify, from the plurality of conditions, at least a first condition and a second condition to be addressed in the user. In some embodiments, the one or more processors can generate the second user interface to include (i) the second plurality of user interface elements for provision of the second digital therapeutic content to address the first condition and (ii) a third plurality of user interface elements for provision of third digital therapeutic content to address the second condition.

In some embodiments, the one or more processors can receive input data identifying the interactions by the user with the first plurality of user interface elements of the first user interface. In some embodiments, the one or more processors can determine a change in state associated with the user based on the input data on the first user interface. In some embodiments, the one or more processors can generate the second user interface responsive to determining the change in state.

In some embodiments, the one or more processors can select, from a plurality of user interface sets, a user interface set comprising at least a portion of the second plurality of user interface elements based on input data identifying the interactions by the user with the first plurality of user interface elements of the first user interface. In some embodiments, the one or more processors can generate the second user interface to include at least the portion of the second plurality of user interface elements defined by the user interface set. In some embodiments, the one or more processors can apply a machine learning model to the input data to select the user interface set from the plurality of user interface sets, wherein the machine learning model is establishing using a plurality of selections each identifying sample input data and a respective user interface set selected from the plurality of user interface sets.

In some embodiments, the one or more processors can receive input data identifying the interactions by the user with the second plurality of user interface elements of the second user interface. In some embodiments, the one or more processors can identify a change in state associated with the user based on the input data on the second user interface. In some embodiments, the one or more processors can determine, responsive to the change in state, to provide the first user interface comprising the first plurality of user interface elements to present the first digital therapeutic content to the user. In some embodiments, the one or more processors can receive, from a remote computing system, an indication to provide a digital therapeutic associated with the second digital therapeutic content to the user to address the condition.

In some embodiments, the plurality of conditions can include at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease. In some embodiments, the user may be on a medication to address the condition, in partial concurrence with the presentation of at least one of the first digital therapeutic content or the second digital therapeutic content.

### DESCRIPTION OF DRAWINGS

FIG. 1 is a schematic view of a system for treating symptoms associated with multiple indications including an indication-agnostic prescription digital therapeutic in accordance with an exemplary embodiment of the present disclosure;
FIG. 2 is a schematic view of example components of the indication-agnostic prescription digital therapeutic of FIG. 1;
FIG. 3 is a schematic view of example components of the indication-agnostic prescription digital therapeutic of FIG. 1;
FIGS. 4A-4C are exemplary GUIs of the indication-agnostic prescription digital therapeutic of FIG. 1 displayed on a display of a patient electronic device;
FIG. 5 is a flowchart illustrating a method for implementing an indication-agnostic prescription digital therapeutic configured to treat symptoms associated with multiple indications, in accordance with an exemplary embodiment of the present disclosure;
FIG. 6 is a flowchart depicting a process of generating user interfaces based on conditions associated with users, in accordance with an illustrative embodiment; and
FIG. 7 is a schematic view of an example computing device that may be used to implement the systems and methods described herein.

Like reference symbols in the various drawings indicate like elements

### DETAILED DESCRIPTION

Example embodiments are provided so that this disclosure will be thorough, and will fully convey the scope to those who are skilled in the art. Numerous specific details are set forth such as examples of specific compositions, components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

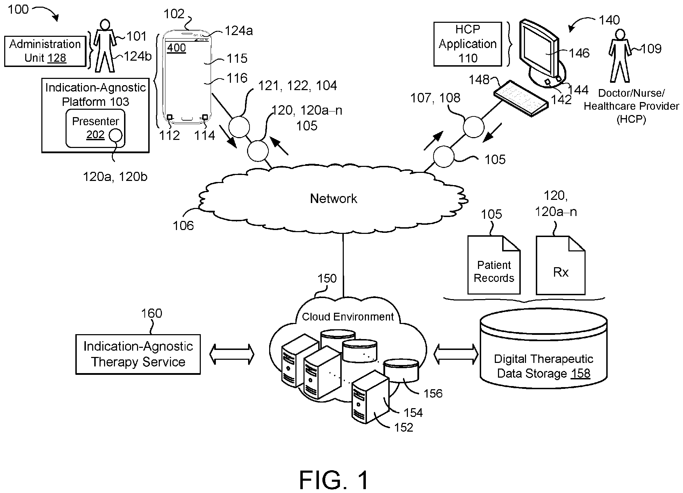

Referring to FIG. 1, in some implementations, a therapy prescription system 100 provides a patient 101 access to one or more prescription digital therapeutics 120, 120a-n (also referred to as prescription digital therapeutics 120 and digital therapeutics 120) prescribed to the patient 101 and monitors events associated with the patient's 101 interaction with the prescription digital therapeutic 120. The prescription digital therapeutics 120 may include both indication-agnostic digital therapies 120 capable of being administered to treat multiple indications, and indication-specific digital therapies 120 administered to treat a specific indication. Although the digital therapeutics 120 are described herein as being a "prescription" digital therapeutics, it is understood that, according to some implementations, the digital therapeutics 120 will not require a prescription from a clinician. Rather, in such implementations, the digital therapeutics 120 may be available to a patient without a prescription, and the digital therapeutics 120 nonetheless otherwise functions in accordance with the description of the prescription digital therapeutics 120 described herein. According to implementations in which the digital therapeutics 120 are not prescribed, the person using or being administered the digital therapeutics 120 may be referred to as a "user." A "user" may include a patient 101 or any other person using or being administered the digital therapeutics 120, irrespective of whether the digital therapeutics 120 were prescribed to that person.

As used herein, a digital therapy may also be referred to as a digital-therapeutic configured to deliver evidence-based psychosocial intervention techniques for treating a patient with a particular indication (e.g., disease or disorder), as well as symptoms and/or behaviors associated with the particular disease or disorder. Examples of a particular disease, disorder, or indication include substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer and/or cardiovascular disease or disorder. As one example, the patient 101 may experience one or more disorders and the prescription digital therapeutics 120 may be tailored for addressing one or more symptoms associated with each of the one or more disorders that the patient 101 may experience. As will be discussed in greater detail below, the prescription digital therapeutics 120 may include a single indication-agnostic prescription digital therapeutic platform 103 that can be augmented to administer therapeutic content to treat one or more different indications.

In some implementations, the digital therapeutics 120 may include or be combined with traditional drug therapy, which similarly may or may not require a prescription. An authorized healthcare provider (HCP) 109 (e.g., a doctor, nurse, etc.) may prescribe the patient 101 one or more of the prescription digital therapeutics 120 designed to treat symptoms in the patient 101. The HCP 109 may include a physician, nurse, clinician, or other qualified health professionals. The HCP 109 may provide any suitable level of supervision to the patient 101, including little to no supervision.

In some examples, the system 100 includes a network 106, a patient device 102, an HCP system 140, and an indication-agnostic therapy service 160. The network 106 provides access to cloud computing resources 150 (e.g., distributed system) that execute indication-agnostic therapy service 160 to provide for the performance of services on remote devices. Accordingly, the network 106 allows for interaction between patients 101 and HCPs 109 with the indication-agnostic therapy service 160. For instance, the indication-agnostic therapy service 160 may provide the patient 101 access to the prescription digital therapeutic 120 and receive user input 121 or event data 122 input by the patient 101 associated with the patient's 101 interaction with the prescription digital therapeutics 120. In turn, the indication-agnostic therapy service 160 may store the event data 122 on a storage resource 156.

The network 106 may include any type of network that allows sending and receiving communication signals, such as a wireless telecommunication network, a cellular telephone network, a time division multiple access (TDMA) network, a code division multiple access (CDMA) network, Global system for mobile communications (GSM), a third generation (3G) network, fourth generation (4G) network, fifth generation (5G) network, a satellite communications network, and other communication networks. The network 106 may include one or more of a Wide Area Network (WAN), a Local Area Network (LAN), and a Personal Area Network (PAN). In some examples, the network 106 includes a combination of data networks, telecommunication networks, and a combination of data and telecommunication networks. The patient device 102, the HCP system 140, and the indication-agnostic therapy service 160 communicate with each other by sending and receiving signals (wired or wireless) via the network 106, which, in some examples, may utilize Bluetooth, Wi-Fi, etc. In some examples, the network 106 provides access to cloud computing resources, which may be elastic/on-demand computing and/or storage resources 156 available over the network 106. The term "cloud" services generally refer to a service delivered from one or more remote devices accessible via one or more networks 106, rather than a service performed locally on a user's device.

The patient device 102 may include, but is not limited to, a portable electronic device (e.g., smartphone, cellular phone, personal digital assistant, laptop computer, or wireless tablet device), a desktop computer, or any other electronic device capable of sending and receiving information via the network 106. The patient device 102 includes data processing hardware 112 (a computing device that executes instructions), memory hardware 114, a display 116, and one or more input devices 115 in communication with the data processing hardware 112. In some implementations, the patient device includes a plurality of sensors 124, 124a-n configured to capture user data in addition to the user input 121 received by the indication-agnostic therapy service 160. The plurality of sensors 124 may include first sensor(s) 124a, such as an accelerometer, proximity sensor, an activity or exercise monitor, location sensors such as a global positioning system (GPS), etc., to provide data about the patient 101 and/or the patient's 101 interaction with the patient device 102. Additionally or alternatively, the patient 101 may be connected to second sensor(s) 124b, such as a heart rate sensor or monitor, blood pressure sensor or monitor, a sleep sensor, an activity monitor (e.g., an electrodermal activity monitor), a skin temperature sensor, a sweat monitor, and/or any other suitable sensors or monitors (e.g., wearable sensors or monitors) in communication with the patient device 102. In some implementations, the patient 101 and the patient device 102 may be in communication with an administration unit 128, such as a delivery pump, an injection unit, an implant, an oral absorption unit (e.g., a sublingually dissolvable film or pill), an inhaler, a nasal injector, other transmucosal administration units, etc. For example, the patient device 102 may transmit a recommendation and/or an instruction to the administration unit 128 for a dosage of medication the patient 101 should take. In some examples, the patient device 102 includes the input device, e.g., a keyboard, mouse, microphones, touch sensor behind the display 116, and/or a camera for allowing the patient 101 to input data. The patient device 102 also executes, for display on the display 116 in communication with the data processing hardware 112, a graphical user interface (GUI) 400 configured to capture the user inputs 121 from the patient 101 via the input device(s) 115 (e.g., one or more of a touchscreen, a speech input, an eye gaze input, a gesture, a mouse, trackpad, stylist, etc.) for controlling functionality of the patient device 102. The GUI 400 may be an interface associated with the indication-agnostic platform 103 executing on the patient device 102 that presents a plurality of objects/elements in the GUI 400. The patient device 102 may further include, or be in communication with, an audio output device (e.g., a speaker) that may output audio data to the patient 101. For instance, audible alerts may be output by the speaker to notify the patient 101 about some time sensitive event associated with the one or more prescription digital therapeutics 120. The patient device 102 may also include a physical button disposed on the patient device 102 configured to receive a tactile selection by the patient 101 for invoking the prescription digital therapeutic 120.

In some implementations, the patient device 102 executes the indication-agnostic platform 103 (or accesses a web-based patient application) for establishing a connection with the indication-agnostic therapy service 160 to access the one or more prescription digital therapeutics 120 in response to a prescription 123 being issued for the one or more prescription digital therapeutics 120. For instance, the patient 101 may have access to the indication-agnostic platform 103 for a duration (e.g., 3 months) of each of the prescription digital therapeutics 120 prescribed to the patient 101. Here, the patient device 102 may launch the indication-agnostic platform 103 by initially providing an access code 104 when the prescription digital therapeutic 120 is prescribed by the HCP 109, the access code 104 allowing the patient 101 to access content associated with the prescription digital therapeutics 120 from the indication-agnostic therapy service 160. The content may be specifically tailored for treating/addressing one or more symptoms associated with the one or more specific indications that the patient 101 may be experiencing.

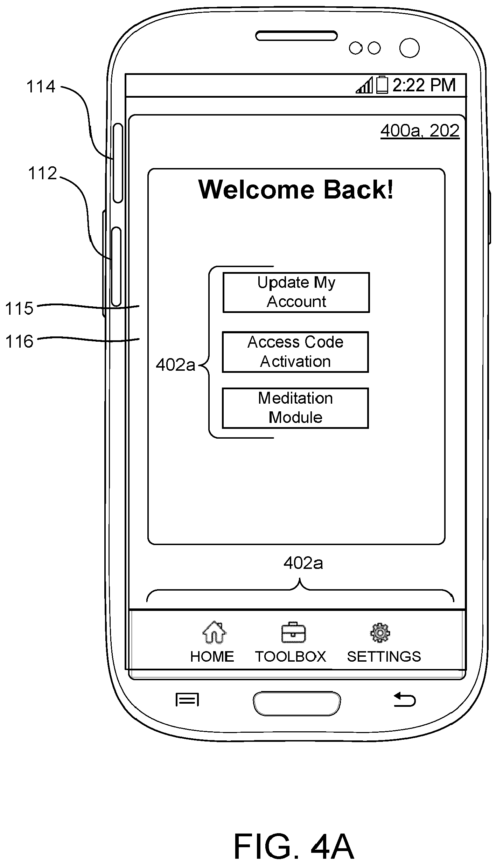

The indication-agnostic platform 103, when executing on the data processing hardware 112 of the patient device 102, is configured to display a variety of GUIs 400 (e.g., the GUIs 400a-c in FIGS. 4A-4C) on the display 116 of the patient device 102 that, among other things, allow the patient 101 to (i) input event data 122 describing one or more parameters associated with the patient 101 (e.g., a signal of how the patient 101 is feeling (e.g., experiencing stress, menstruation, fatigue, sleep-deprivation, etc.)); (ii) solicit information from the patient 101; (iii) deliver therapeutic content (e.g., cognitive behavioral therapy (CBT) content) to the patient 101; (iv) allow the patient 101 to contact their HCP 109; (v) allow the patient 101 to review their progress adhering to their prescription regimen with respect to the prescription digital therapeutics 120 and/or any prescribed medication; and/or (vi) present journal entries for the patient 101 to view and/or edit. According to some examples, the therapeutic content may include textual, auditory, visual, and/or audio/visual content configured to treat one or more symptoms associated with the one or more specific indications that the patient 101 may be experiencing.

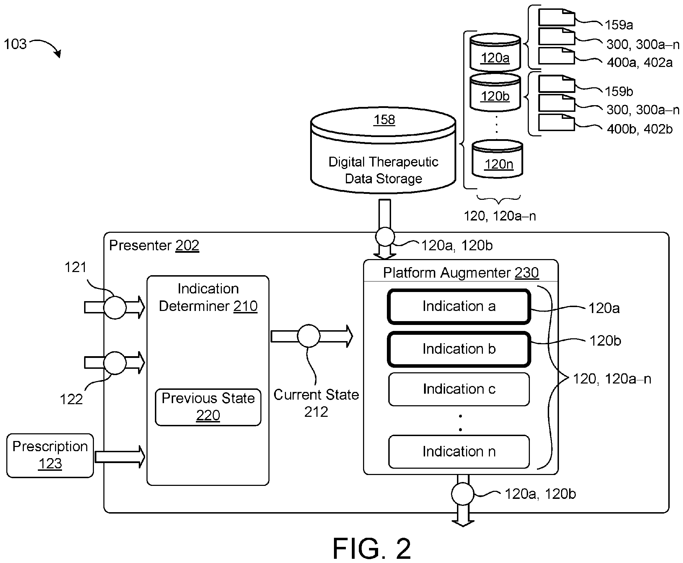

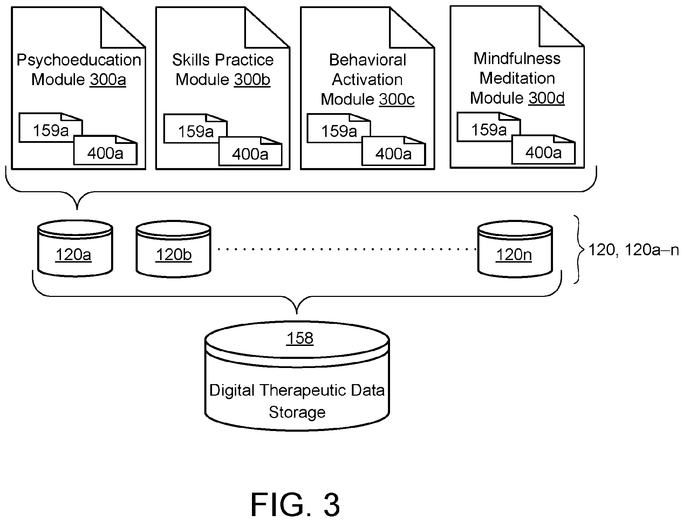

As shown in FIGS. 1 and 2, the storage resources 156 may provide a digital therapeutic database 158 (also referred to as data storage 158 and digital therapeutic data storage 158) for storing the event data 122 received from the patient 101 in a corresponding patient record 105 as well as the one or more prescription digital therapeutics 120 available to the HCP 109 to prescribe for the patient 101. The digital therapeutic database 158 may be a headless content management system (CMS) that stores the one or more prescription digital therapeutics 120 together to simplify the design, development, quality, support, and maintenance costs of the prescription digital therapeutics 120. Each of the prescription digital therapeutics 120 may include indication-specific digital therapeutic content 159, 159a-n and corresponding GUIs 400a-n configured to display graphical elements 402 that the patient 101 may select via user inputs 121 such as touch inputs, speech inputs, eye gaze inputs, gesture inputs, or other input techniques such as via a mouse, stylus, or keyboard. Each of the prescription digital therapeutics 120 in the digital therapeutic database 158 may additionally include one or more modules 300, 300a-n (FIG. 3) for organizing and delivering the indication-specific digital therapeutic content 159 and the corresponding GUIs 400. The one or more modules 300 may deliver the indication-specific digital therapeutic content 159 and the corresponding GUIs 400 in a manner that is tailored to the patient 101 based on the one or more specific indications that the patient 101 may be experiencing. In some implementations, the digital therapeutic database 158 includes third-party information (e.g., from medical databases, from the HCP, peer-reviewed articles, etc.). The third-party information may be assimilated with the indication-specific digital therapeutic content 159 and delivered to the patient 101 via the one or more modules 300 in the corresponding GUIs 400 to treat the one or more indications that the patient may be experiencing. As discussed above, in some implementations, the prescription digital therapeutics 120 include indication-specific digital therapeutics 120 and indication-agnostic digital therapeutics 120. In these implementations, the indication-agnostic digital therapeutics 120 include corresponding GUIs 400, but do not include indication-specific digital therapeutic content 159.

The patient record 105 may be encrypted while stored on the data storage 158 so that any identifying information of the patient 101 is anonymized, but may later be decrypted when the patient 101 or supervising HCP 109 requests the patient record 105 (assuming the requester is authorized/authenticated to access the patient record 105). All data transmitted over the network 106 between the patient device 102 and the cloud computing system 150 may be encrypted and sent over secure communication channels. For instance, the indication-agnostic platform 103 may encrypt the user inputs 121 and the event data 122 before transmitting to the indication-agnostic therapy service 160 via the HTTPS protocol and decrypt a patient record 105 received from the indication-agnostic therapy service 160. When network connectivity is not available, the indication-agnostic platform 103 may store the user inputs 121 and the event data 122 in an encrypted queue within the memory hardware 114 until network connectivity is available.

The HCP system 140 may be located at a clinic, doctor's office, or facility administered by the HCP 109 and includes data processing hardware 142, memory hardware 144, and a display 146. The memory hardware 144 and the display 146 are in communication with the data processing hardware 142. For instance, the data processing hardware 142 may reside on a desktop computer or portable electronic device for allowing the HCP 109 to input and retrieve data to and from the indication-agnostic therapy service 160. In some examples, the HCP 109 may initially onboard some or all of patient data 107 at the time of prescribing the prescription digital therapeutic 120 to the patient 101. The HCP system 140 includes a keyboard 148, mouse, microphones, speakers and/or a camera.

In some implementations, the HCP system 140 (i.e., via the data processing hardware 142) executes the HCP application 110 (or accesses a web-based patient application) for establishing a connection with the indication-agnostic therapy service 160 to input and retrieve data therefrom. For instance, the HCP system 140 may be able to access the anonymized patient record 105 securely stored by the indication-agnostic therapy service 160 on the storage resources 156 by providing an authentication token 108 validating that the HCP 109 is supervising the patient 101 and authorized to access the corresponding patient record 105. The authentication token 108 may identify the particular patient 101 associated with the patient record 105 that the HCP system 140 is permitted to obtain from the indication-agnostic therapy service 160. The patient record 105 may include timestamps with the event data 122 indicating the patient's interaction with the prescription digital therapeutics 120 through the indication-agnostic platform 103 executing on the patient device 102. The HCP application 110, when executing on the data processing hardware 142 of the HCP system 140, is configured to display a variety of graphical user interfaces (GUIs) on the display 146 of the HCP system 140 that, among other things, allow the HCP 109 to input event data 122 describing one or more parameters associated with the patient 101, solicit information from the patient 101, and input clinical notes associated with the patient 101.

In some implementations, the HCP application 110 is in communication with a single indication-agnostic platform 103 for a single patient 101 and manages data associated with the single indication-agnostic platform 103 while simultaneously managing data associated with each of the prescription digital therapeutics 120 in the single indication-agnostic platform 103. In other implementations, the HCP application 110 is in communication with several indication-agnostic platforms 103 associated with several patients 101, and the HCP application 110 may manage and display the data associated with the several indication-agnostic platforms 103 for each patient 101 in a group of patients 101 in any suitable manner, e.g., by toggling between different views and/or displaying certain data simultaneously. In these implementations, the data from the several indication-agnostic platforms 103 may be displayed simultaneously in any suitable manner or the data from each indication-agnostic platform 103 may be displayed discretely such that the HCP 109 is able to toggle between the discretely displayed data for the several patients 101.

The cloud computing resources 150 may be a distributed system (e.g., remote environment) having scalable/elastic resources 152. The resources 152 include computing resources 154 (e.g., data processing hardware) and/or the storage resources 156 (e.g., memory hardware). The cloud computing resources 150 execute the indication-agnostic therapy service 160 for facilitating communications with the patient device 102 and the HCP system 140 and storing data on the storage resources 156 within the data storage 158. In some examples, the indication-agnostic therapy service 160 and the data storage 158 reside on a standalone computing device. The indication-agnostic therapy service 160 may provide the patient 101 with the indication-agnostic platform 103 (e.g., a mobile application, a web-site application, or a downloadable program that includes a set of instructions) executable on the data processing hardware 112 and accessible through the network 106 via the patient device 102 when the patient 101 provides a valid access code 104. Similarly, the indication-agnostic therapy service 160 may provide the HCP 109 with the HCP application 110 (e.g., a mobile application, a web-site application, or a downloadable program that includes a set of instructions) executable on the data processing hardware 142 and accessible through the network 106 via the HCP system 140.

Referring to FIG. 2, the indication-agnostic platform 103 includes a presenter 202 which manages which of the prescription digital therapeutics 120 are made available (i.e., presented) to the patient 101. That is, the indication-agnostic platform 103 controls which of the prescription digital therapeutics 120 including corresponding GUIs 400 and digital therapeutic content 159 delivered by one or more modules 300a-n are presented as available options to the patient 101 while the patient 101 has access to the indication-agnostic platform 103. In this sense, the presenter 202 (e.g., via the GUI 400), is configured to present the digital therapeutic content 159 in the one or more modules 300 on the user device 102 for the corresponding indication-specific prescription digital therapeutics 120. Advantageously, the indication-agnostic platform 103 allows the patient 101 to navigate between the one or more prescription digital therapeutics 120 from the available prescription digital therapeutics 120 using the GUI 400.

The presenter 202 may include an indication determiner 210 and a platform augmenter 230. The indication determiner 210 may be configured to obtain at least one of the user inputs 121 and the event data 122 and analyze the user input data 121 and/or the event data 122 to determine a current state 212 associated with one or more indication-specific prescription digital therapeutics 120 that may be therapeutic for the patient 101 and generates, as output, the current state 212. In some implementations, the indication determiner 210 generates the current state 212 in response to receiving a prescription 123 issued for an indication-specific prescription digital therapeutic 120. The platform augmenter 230 may, based on the current state 212, access the digital therapeutic data storage 158 storing all of the prescription digital therapeutics 120 that the indication-agnostic prescription digital therapeutic platform 103 is equipped to present to the patient 101 via the GUI 400 to obtain the one or more indication-specific prescription digital therapeutics 120. In the example shown in FIG. 2, the platform augmenter 230 accesses the digital therapeutic data storage 158 and obtains the prescription digital therapeutics 120a, 120b. The platform augmenter 230 then augments the indication-agnostic prescription digital therapeutic platform 103 to include the indication-specific prescription digital therapeutics 120a, 120b.

In some implementations, the indication determiner 210 maintains a record of a previous state 220 associated with a prescription digital therapeutic 120 available on the indication-agnostic prescription digital therapeutic platform 103. For example, the current state 220 of the indication-agnostic prescription digital therapeutic platform 103 may include an indication-agnostic prescription digital therapeutic 120. In this example, after receiving the user input data 121 and/or the event data 122, the indication determiner 210 may determine that the previous state 220 does not include the one or more indication-specific prescription digital therapeutics 120 associated with the current state 212 that may be therapeutic for the patient 101. In other words, the indication determiner 210 may determine that the indication-agnostic prescription digital therapeutic 120 of the previous state 220 is different than the indication-specific prescription digital therapeutics 120 of the current state 212. This change between the previous state 220 and the current state 212 triggers the platform augmenter 230 to augment the indication-agnostic prescription digital therapeutic platform 103 to include the indication-specific prescription digital therapeutics 120 associated with the current state 212. In some examples, however, the indication determiner 210 may determine that the indication-specific prescription digital therapeutics 120 associated with the current state 212 are the same as the indication-agnostic prescription digital therapeutic 120 associated with the previous state 220, the indication determiner 210 may not send the current state 212 to the platform augmenter 230.

In some examples, the indication determiner 210 only outputs the current state 212 to the platform augmenter 230 (thereby triggering the platform augmenter 230) when there is a difference between the previous state 220 and the current state 212. For instance, the indication determiner 210 may be configured with an indication change threshold and, when the difference detected between the previous state 220 and the current state 212 satisfies the indication change threshold (e.g., exceeds the threshold), the state determiner 210 outputs the current state 212 to the platform augmenter 230. The threshold may be zero, where the slightest difference between the previous state 220 and the current state 212 detected by the indication determiner 210 may trigger the platform augmenter 230 of the presenter 202 to augment the indication-agnostic prescription digital therapeutic platform 103. Conversely, the threshold may be higher than zero to prevent unnecessary augmentation of the indication-agnostic prescription digital therapeutic platform 103 by the platform augmenter 230 as a type of platform-interruption sensitivity mechanism.

Referring to FIG. 3, the indication-specific prescription digital therapeutic 120a may include one or more modules 300a-d each configured to deliver the indication specific graphical content 159a and the corresponding GUIs 400a tailored to the patient 101. In the example, the indication-specific therapeutic content 120a includes a "Psychoeducation" module 300a, a "Skills Practice" module 300b, a "Behavioral Activation" module 300c, and a "Mindfulness Meditation" module 300d. The modules 300a-d do not represent an exhaustive list, but rather are an exemplary list of modules 300 that may be included as part of the indication-specific prescription digital therapeutic 120a. For example, the HCP 109 may activate additional modules 300 and/or indication-specific graphical content 159 that is specific to the needs of the patient 101. In these examples, the HCP 109 may push the additional modules 300 to the indication-agnostic prescription digital therapeutic platform 103 such that, when the patient 101 reenters the access code 104, the additional modules 300 are available to the patient 101. In other examples, the indication-agnostic prescription digital therapeutic platform 103 includes Artificial Intelligence (AI) that analyzes the user inputs 121 and/or patterns in the patient 101 use of the indication-agnostic prescription digital therapeutic platform 103 and generates recommendations for additional modules 300 to the HCP 109 that may be beneficial to the patient 101. In these examples, the additional modules 300 to the indication-agnostic prescription digital therapeutic platform 103 may be added if the HCP 109 approves the recommendations by the AI.

Alternatively, in some implementations, the digital therapeutic database 158 stores one or more modules 300, 300a-n, where each module 300 includes one or more indication-specific prescription digital therapeutics 120 with corresponding indication-specific graphical content 159. In other words, each module 300 includes the indication-specific graphical content 159 for one or more indication-specific prescription digital therapeutics 120, such that the module 300 may pool/share any indication-specific graphical content 159 that is common across the one or more indication-specific prescription digital therapeutics 120. For example, the "Skills Practice" module 300b may include indication-specific graphical content 159a for breathing exercises corresponding to a first indication-specific prescription digital therapeutics 120a, as well as indication-specific graphical content 159b for breathing exercises corresponding to a second indication-specific prescription digital therapeutics 120b.

Referring to FIGS. 4A-4C, schematic views of exemplary GUIs 400a-c of the prescription digital therapeutics 120 (e.g., by execution of the indication-agnostic prescription digital therapeutic platform 103) displayed on the display 116 of the patient device 102 for treating symptoms associated with one or more indications. The example GUIs 400a-c are configured to display the indication-specific graphical content 159 including one or more graphical elements that the patient 101 may select via the input device(s) 115. As discussed above with respect to FIG. 3, the indication-specific graphical content 159 may be delivered in one or more modules 300, where the indication-specific graphical content 159 is tailored to the patient 101 based on the one or more specific indications that the patient 101 may be experiencing.

Referring to FIG. 4A, in some implementations, upon launching the indication-agnostic prescription digital therapeutic platform 103, the indication-agnostic prescription digital therapeutic platform 103 displays a first GUI 400a including first graphical user interface elements 402a associated with a prescription digital therapeutic 120a. As shown, the GUI 400a includes a welcome screen for the patient 101 and provides first graphical user interface elements 402a that allow the patient 101 to update an account of the patient 101, activate a prescription digital therapeutic 120, or access an indication-agnostic digital therapeutic 120 such as a meditation module. In other words, the indication-agnostic prescription digital therapeutic platform 103 does not include indication-specific digital therapeutic content 159. The first graphical user interface elements 402a may further include elements for the patient 101 to navigate within the indication-agnostic prescription digital therapeutic platform 103 (i.e., between one or more prescription digital therapeutics 120).

In some examples, the HCP 109 issues a prescription 123 for a first indication-specific prescription digital therapeutic 120b. As discussed with respect to FIG. 1 above, the patient 101 may receive an access code 104 when the prescription digital therapeutic 120b is prescribed by the HCP 109. The patient 101 may enter the access code 104 in the first graphical user interface element 402a to access the first indication-specific prescription digital therapeutic 120b. As discussed above with reference to FIG. 2, the first indication-specific prescription digital therapeutic 120b may include a second GUI 400b and first indication-specific digital therapeutic content 159b. In response to the prescription 123 being issued for the first indication-specific prescription digital therapeutic 120b, the presenter 202 (via the augmenter 230) augments the indication-agnostic prescription digital therapeutic platform 103 to include the first indication-specific prescription digital therapeutic 120b. In other words, the indication-agnostic prescription digital therapeutic platform 103 is augmented to include the second GUI 400b associated with the first indication-specific prescription digital therapeutic 120b in addition to the first GUI 400a.

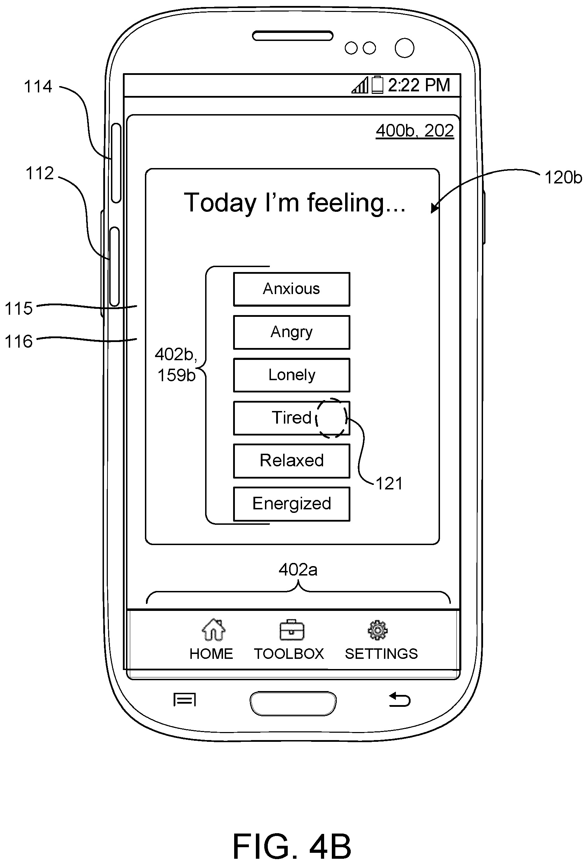

Referring now to FIG. 4B, the indication-agnostic prescription digital therapeutic platform 103 displays the second GUI 400b of the first indication-specific prescription digital therapeutic 120b. In some implementations, the indication-agnostic prescription digital therapeutic platform 103 displays the second GUI 400b in response to the patient 101 entering the access code 104 in the first graphical user interface element 402a of FIG. 4A for the first indication-specific prescription digital therapeutic 120b. As shown, the first indication-specific prescription digital therapeutic 120b may treat symptoms associated with generalized anxiety disorder (GAD), where the presenter 202 (via the indication-agnostic prescription digital therapeutic platform 103) administers the first indication-specific digital therapeutic content 159b of the first indication-specific prescription digital therapeutic 120b via the second GUI 400b to treat GAD. Here, the second GUI 400b may display the first graphical user interface elements 402a that allow the patient 101 to navigate within the indication-agnostic prescription digital therapeutic platform 103 in addition to second graphical user interface elements 402b.

In the example shown, the second GUI 400b provides the second graphical user elements 402b, where each of the second graphical user elements 402b is associated with a corresponding feeling of the patient 101. The second graphical user elements 402b may be prepopulated based on common feelings a typical patient diagnosed with GAD may be experiencing. The second GUI 400b allows the patient 101 to input a particular feeling they are presently experiencing, or has recently experienced. In the example, the second graphical user elements 402b include an "Anxious" element 402b, an "Angry" element 402b, a "Lonely" element 402b, a "Tired" element 402b, a "Relaxed" element 402b, and an "Energized" element 402b. The second graphical user elements 402b do not represent an exhaustive list, but rather are an exemplary list of elements 402b that may be included as part of the second GUI 400b.

While administering the first indication-specific digital therapeutic content 159b, the patient device 102 detects a user input 121 corresponding to the "Tired" element 402b indicating the patient 101 is feeling tired. Here, the indication determiner 210 (FIG. 2) obtains the user input 121 and analyzes the user input 121 to determine a second indication-specific prescription digital therapeutic 120c that is different than the first indication-specific prescription digital therapeutic 120b. In response to determining a difference between the first indication-specific prescription digital therapeutic 120b (i.e., the previous state 220) and the second indication-specific prescription digital therapeutic 120c (i.e., the current state 212) the platform augmenter 230 augments the indication-agnostic prescription digital therapeutic platform 103 to include the second indication-specific prescription digital therapeutic 120c. In other words, the indication-agnostic prescription digital therapeutic platform 103 is augmented to include the third GUI 400c associated with the second indication-specific prescription digital therapeutic 120c in addition to the second GUI 400b associated with the first indication-specific prescription digital therapeutic 120b and the first GUI 400a.

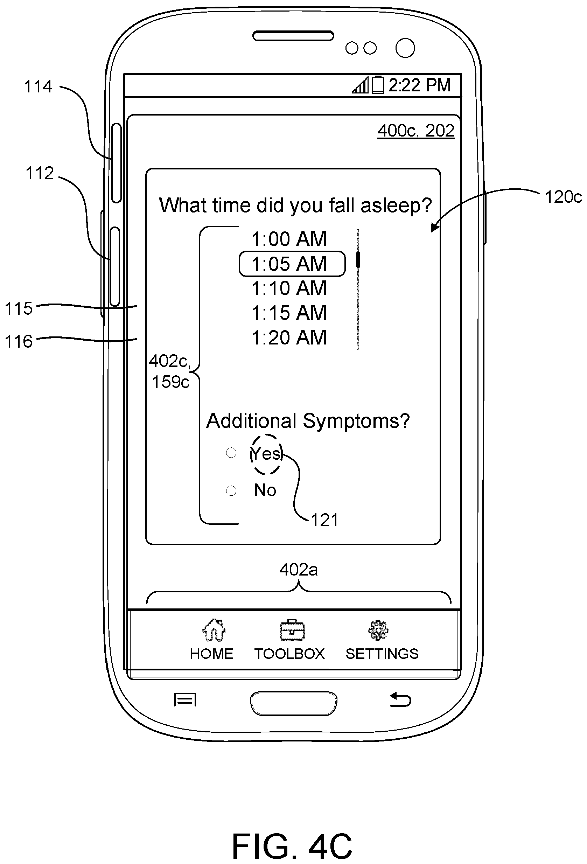

In the example shown in FIG. 4C, in response to detecting the user input 121 corresponding to the "Tired" element 402b indicating the patient 101 is feeling tired and determining that there is a difference between the previous state 220 and the current state 212, the indication-agnostic prescription digital therapeutic platform 103 displays the third GUI 400c of the second indication-specific prescription digital therapeutic 120c. As discussed above with reference to FIG. 2, the second indication-specific prescription digital therapeutic 120c may include the third GUI 400c and second indication-specific digital therapeutic content 159c. Here, the second indication-specific digital therapeutic content 159 of the second indication-specific prescription digital therapeutic 120c may treat symptoms associated with insomnia, where the presenter 202 (via the indication-agnostic prescription digital therapeutic platform 103) administers the second indication-specific digital therapeutic content 159c of the second indication-specific prescription digital therapeutic 120c via the third GUI 400c to treat insomnia. The third GUI 400c may display the first graphical user interface elements 402a that allow the patient 101 to navigate within the indication-agnostic prescription digital therapeutic platform 103 in addition to third graphical user interface elements 402c.

In the example shown, the third GUI 400c provides the third graphical user elements 402c, where each of the third graphical user elements 402c allow the patient 101 to identify a time when symptoms of insomnia set in, as well as an input to identify additional symptoms by providing a user input 121. In the example, the third graphical user elements 402c additionally include an "Additional Symptoms?" element 402c, a "Yes" element 402c, and a "No" element 402c. In some implementations, while administering the second indication-specific digital therapeutic content 159c, the patient device 102 detects a user input 121 corresponding to the "Yes" element 402c indicating the patient 101 is experiencing additional symptoms. In these implementations, the indication-agnostic prescription digital therapeutic platform 103 may display the second GUI 400b to obtain additional user input. In other words, one or more of the graphical user elements 402a-c may allow the patient 101 to toggle between prescription digital therapeutics 120a-c available on the indication-agnostic prescription digital therapeutic platform 103.

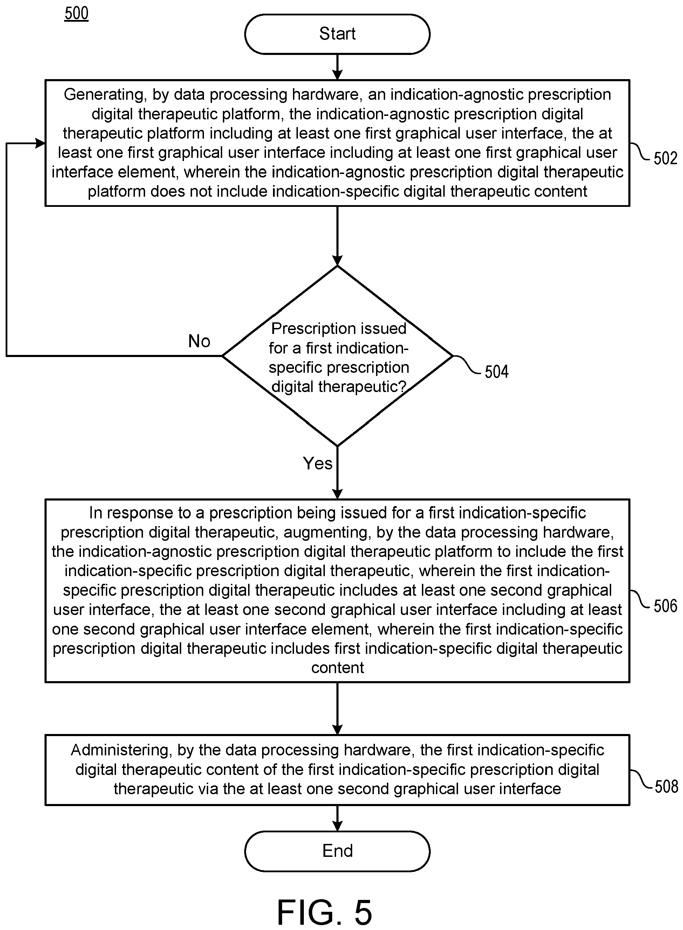

Referring to FIG. 5, a flowchart illustrating a method 500 for implementing the prescription digital therapeutic 120 is generally shown. The method 500 may include a treatment of at least one indication. The method 500 includes, at operation 502, generating, by data processing hardware 112, an indication-agnostic prescription digital therapeutic platform 103 including at least one first graphical user interface 400, 400a. Here, the at least one first graphical user interface 400a includes at least one first graphical user element 402, 402a, and the indication-agnostic prescription digital therapeutic platform 103 does not include indication-specific digital therapeutic content 120.

At operation 504, the method 500 also includes determining whether a prescription 123 has been issued for a first indication-specific prescription digital therapeutic 120. If the indication-agnostic prescription digital therapeutic platform 103 determines, at operation 504, that a prescription 123 has not been issued for a first indication-specific prescription digital therapeutic 120, then the indication-agnostic prescription digital therapeutic platform 103 is configured to return to 502, where the treatment is continued. If the indication-agnostic prescription digital therapeutic platform 103 determines, at 504, that a prescription 123 has been issued for a first indication-specific prescription digital therapeutic 120, then the indication-agnostic prescription digital therapeutic platform 103 is configured to proceed to operation 506.

At operation 506, the method 500 includes, in response to the prescription 123 being issued for a first indication-specific prescription digital therapeutic 120, 120a, augmenting, by the data processing hardware 112, the indication-agnostic prescription digital therapeutic platform 103 to include the first indication-specific prescription digital therapeutic 120a. Here, the first indication-specific prescription digital therapeutic 120a includes at least one second graphical user interface 400, 400b. The at least one second graphical user interface 400b includes at least one second graphical user interface element 402, 402b, where the first indication-specific prescription digital therapeutic 120a includes first indication-specific digital therapeutic content 159, 159a. The method 500 further includes, at operation 508, administering, by the data processing hardware 112, the first indication-specific digital therapeutic content 159a of the first indication-specific prescription digital therapeutic 120a via the at least one second graphical user interface 400b. It should be understood that the method 500 may include additional or fewer steps than those shown and described, and certain steps may be omitted or performed in any suitable order.

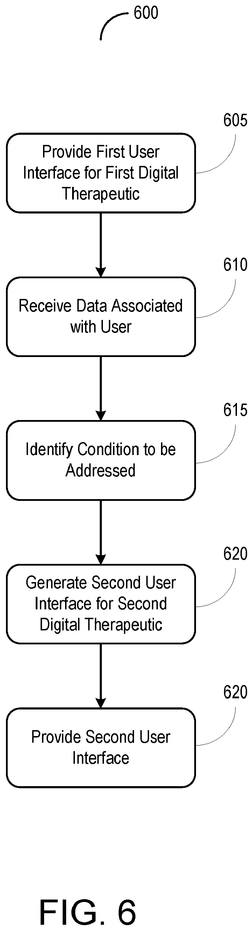

FIG. 6 is a flowchart depicting a process 600 of generating user interfaces based on conditions associated with users. The process 600 can be implemented or performed by any of the components detailed herein, such as the data processing hardware 112, 142, 154 or a computing device 700, among others. The process 600 can include any of the operations detailed herein. In brief overview, under the process 600, a computing system can provide a first user interface for a first digital therapeutic (605). The computing system can receive data associated with a user (610). The computing system can identify a condition to be addressed (615). The computing system can generate a second user interface for a second digital therapeutic (620). The computing system can provide the second user interface (625).

In further detail, a computing system (e.g., the data processing hardware 112, 142, 154, or the computing device 700) can present or provide a first user interface (e.g., GUIs 400) for a first digital therapeutic content (e.g., the indication-agnostic digital therapeutics 120) to a user (e.g., the patient 101) (605). The first user interface can include a first set of user interface elements to display, render, or otherwise present the first digital therapeutic content to the user. The first user interface can be presented to the user via an application (e.g., digital therapeutic platform 103) executing on an end user device (e.g., the patient device 102). The first user interface can be used by the user to address a set of conditions (also referred herein as indications) via interactions with first set of user interface elements. The first user interface may not be particular or specific to a given condition. The digital therapeutic content for the first user interface may be employed across different indications that are similar but not identical, taking advantage of the core treatment as a starting point or template. For example, health conditions such as fatigue and nausea are suffered by users of multiple diseases like cancer and pregnancy. As another example, the same digital therapeutic content to treat major depressive disorder may be used in depression settings in users with breast cancer.

The computing system can retrieve, identify, or otherwise receive data (e.g., input data 121, event data 122, or prescription 123) associated with the user (610). In some embodiments, the computing system can receive input data (e.g., the input data 121 or the event data 122) identifying interactions by the first set of user interface elements of the first user interface. The interactions may be with the first digital therapeutic content provided via the first set of user interface elements of the first user interface. In some embodiments, the computing system can retrieve, obtain, or otherwise receive an indication (e.g., the prescription 123) to provide a second digital therapeutic content (e.g., indication-specific digital therapeutic 120) to address a particular condition (e.g., a disease, disorder, or other indication) from a remote computing device (e.g., the HCP 109). The indication can correspond to a prescription issued by an entity associated with the remote computing device to address the particular condition of the user. The indication can reference, define, or otherwise identify a particular second digital therapeutic for the specific condition to be provided to the user. The indication can be issued while the user is being provided the indication-agnostic digital therapeutic.

The computing system can determine, select, or otherwise identify at least one condition to be addressed in the user using the data associated with the user (615). The conditions may be identified or selected from a set of conditions. The conditions may include, for example, substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease, among others. In at least partial concurrence with the digital therapeutic content (e.g., provided via user interfaces), the user may be on a medication (e.g., traditional drug therapy) to address the condition. In some embodiments, the computing system can identify multiple conditions (e.g., first and second conditions) to be addressed in the user, from the set of the conditions based on the data associated with the user.

In some embodiments, the computing system can identify the condition based on the input data received via the first set of user interface elements of the first user interface. Using the input data, the computing device can track, determine, or identify a state (e.g., the previous state 220 or current state 212) associated with the user or the first digital therapeutic content. The state may indicate or identify, for example, a degree of severity of a condition within the user or emotional state of the user, among others. In some embodiments, the computing system can identify, detect, or otherwise determine whether a change in the state has occurred based on the input data. The change can correspond to a difference from the previous state (e.g., the previous state 220) to the current state (e.g., the current state 212). When the change in the state has been determined, the computing system can identify the condition associated with the change. For example, when the user indicates a change in emotion from a normal state to a tired state, the computing system can identify the condition associated with the tired state.

In some embodiments, the computing system can identify the condition based on the indication of the digital therapeutic content to be issued from the remote computing device. The indication can correspond to a issuance of the prescription for a specific condition for the user. Upon receipt of the indication, the computing system can process or parse the indication to extract or identify the specific condition for the user. In some embodiments, the computing system can determine or identify the condition based on the indication-specific digital therapeutic specified by the indication from the remote computing device.

The computing system can produce, create, or otherwise generate a second user interface for the second digital therapeutic content (620). The second user interface can be generated by the computing system to identify or include: at least a portion of the first set of user interface elements; and a second set of user interface elements to provide or present the second digital therapeutic content (e.g., indication-specific digital therapeutic 120) to address the identified condition. In some embodiments, the computing system can generate the second user interface in response to determination of the change in state. In some embodiments, the computing system can generate the second user interface to include: one set of user interface elements for the provision of one digital therapeutic content for one identified condition; and another set of user interface elements for the provision of another digital therapeutic content for another identified condition, among others.

In some embodiments, in generating the second user interface element, the computing system can identify or select at least one user interface set from a plurality of user interface sets (e.g., modules 300). Each user interface set can identify or include one or more user interface elements to be included in the second set of user interface elements in the second user interface for providing the second digital therapeutic content. Each user interface set can include at least a portion of the indications-specific digital therapeutic content (e.g., indication-specific graphical content 159) for the identified condition to be addressed in the user. The selection of the user interface set can be based on the input data identifying interactions with the first set of user interface elements. The selection can be also based on the identification of the condition to be addressed in the user. With the selection, the computing system can generate the second user interface to include the user interface set as a portion of the second set of user interface elements.

In some embodiments, the computing system can identify or select the user interface set from the plurality of user interface sets by applying a machine learning (ML) model (e.g., artificial intelligence (AI) of the digital therapeutic platform 103) to the input data. The ML model can initialized, trained, or otherwise established using a training dataset including a set of example selections. Each example can include or identify sample input data (e.g., identifying interactions with indication-agnostic digital therapeutic) and a selected user interface set for the input data. The ML model can be of AI architecture, for example, an artificial neural network (ANN), a clustering model (e.g., a k-nearest neighbors clustering model), a support vector machine (SVM), a random forest, or a decision tree, among others. From applying the ML model to the input data as input, the computing system can identify or select the user interface set from the output of the ML model.

The computing system can present or provide the second user interface (e.g., GUIs 400) including the second set of user interface element for the second digital therapeutic content to the user to address the identified condition (625). The second user interface can include the second set of user interface elements to display, render, or otherwise present the second digital therapeutic content to the user. The second user interface can be presented to the user via the application executing on the end user device. The second user interface can be used by the user to address the identified condition via interactions with second set of user interface elements. The second user interface may be particular or specific to a given condition (e.g., the identified condition). For example, a user may be initially provided with a digital therapeutic to address itching or scratching symptoms for atopic dermatitis or psoriasis. However, with additional data indicating an excessive amount of fatigue as a new symptom, the computing system may augment the user interfaces provided to the user.

The computing system can repeat the process 600 for the second user interface as detailed herein above with respect to the first user interface. In some embodiments, the computing system can retrieve, identify, or otherwise receive data (e.g., input data 121 or event data 122) associated with the user, subsequent to the provision of the second user interface. In some embodiments, the computing system can receive input data (e.g., the input data 121 or the event data 122) identifying interactions by the second set of user interface elements of the second user interface. The interactions may be with the second digital therapeutic content provided via the second set of user interface elements of the second user interface. In some embodiments, the computing system can retrieve, obtain, or otherwise receive an indication (e.g., the prescription 123) to provide a third digital therapeutic content (e.g., indication-specific digital therapeutic 120) to address another condition (e.g., a disease, disorder, or other indication) from the remote computing device (e.g., the HCP 109).

The computing system can determine, select, or otherwise identify at least one second condition to be addressed in the user using the data associated with the user, in conjunction with the provision of the second digital therapeutic content. In some embodiments, the computing system can identify the second condition based on the input data received via the second set of user interface elements of the second user interface. Using the input data, the computing device can track, determine, or identify the state associated with the user or the second digital therapeutic content. In some embodiments, the computing system can identify, detect, or otherwise determine whether a change in the state has occurred based on the input data associated with the second digital therapeutic content. When the change in the state has been determined, the computing system can identify the second condition associated with the change. The change in state may be to the original state associated with the first user interface or to another state for another condition. For instance, a user with breast cancer may be presented with the second user interface to provide digital therapeutic for fatigue as a symptom of breast cancer. When the data indicates insomnia in the user, the computing system can augment the user interfaces for the user with digital therapeutic content to address insomnia.

In some embodiments, the computing system can identify the second condition based on the indication of the digital therapeutic content to be issued from the remote computing device. The indication can correspond to an issuance of the prescription for another condition for the user. Upon receipt of the indication, the computing system can process or parse the indication to extract or identify the specific condition for the user. In some embodiments, the computing system can determine or identify the second condition based on the indication-specific digital therapeutic specified by the indication from the remote computing device.

In some embodiments, the computing system can generate a third user interface to identify or include: at least a portion of the second user interface; and a third set of user interface elements to present or provide a third digital therapeutic content to address the second condition. The generation of the third user interface can be similar as the generation of the second user interface as detailed herein. In some embodiments, the computing system can determine to provide the first user interface to present or provide the indication-agnostic digital therapeutic content to the user, when the change in the state is back to the original state. The computing system can repeat the process 600 as detailed herein any number of times.

In this manner, the computing system can dynamically and automatically generate user interfaces to provide digital therapeutic content to address various identified conditions in the user. The computing system may be able to provide digital therapeutic content for a variety of conditions through at least one application, thereby reducing computational storage overhead and burden relative to maintaining individual applications for respective conditions. By providing the user interfaces based on data associated with the user, the computing system can improve the quality of human-computer interactions (HCI) between the user and the digital therapeutic platform. The dynamic and automated generation of user interfaces can increase adherence of the user to a digital therapeutic regimen. Relative to static approaches for providing user interfaces to administer digital therapeutics, the computing system can reduce or decrease consumption of computing resources (e.g., processing power and memory) as well as network bandwidth from providing ineffective, static digital therapeutics content. Furthermore, the computing system can improve the efficacy of the medication taken by the user in alleviating or treating the condition.

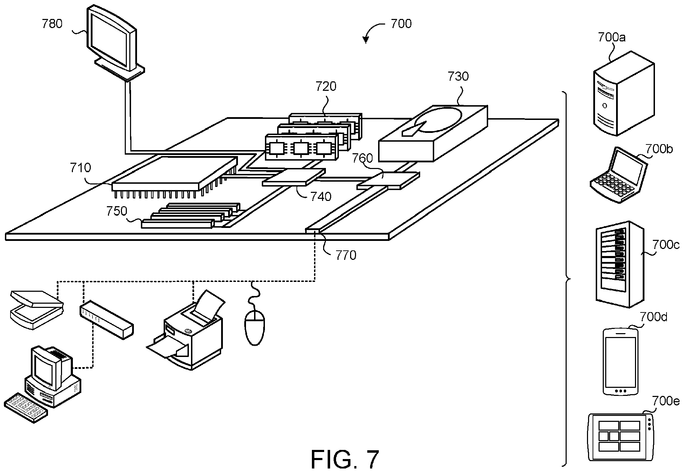

FIG. 7 is schematic view of an example computing device 700 that may be used to implement the systems and methods described in this document. The computing device 700 is intended to represent various forms of digital computers, such as laptops, desktops, workstations, personal digital assistants, servers, blade servers, mainframes, and other appropriate computers. The components shown here, their connections and relationships, and their functions, are meant to be exemplary only, and are not meant to limit implementations of the inventions described and/or claimed in this document.

The computing device 700 includes a processor 710 (e.g., data processing hardware 112, 142, 154 of FIG. 1), memory 720 (e.g., memory hardware 114, 144, 156 of FIG. 1), a storage device 730, a high-speed interface/controller 740 connecting to the memory 720 and high-speed expansion ports 750, and a low speed interface/controller 760 connecting to a low speed bus 770 and a storage device 730. Each of the components 710, 720, 730, 740, 750, and 760, are interconnected using various busses, and may be mounted on a common motherboard or in other manners as appropriate. The processor 710 can process instructions for execution within the computing device 700, including instructions stored in the memory 720 or on the storage device 730 to display graphical information for a graphical user interface (GUI) on an external input/output device, such as display 780 coupled to high speed interface 740. In other implementations, multiple processors and/or multiple buses may be used, as appropriate, along with multiple memories and types of memory. Also, multiple computing devices 700 may be connected, with each device providing portions of the necessary operations (e.g., as a server bank, a group of blade servers, or a multi-processor system).

The memory 720 stores information non-transitorily within the computing device 700. The memory 720 may be a computer-readable medium, a volatile memory unit(s), or non-volatile memory unit(s). The non-transitory memory 720 may be physical devices used to store programs (e.g., sequences of instructions) or data (e.g., program state information) on a temporary or permanent basis for use by the computing device 700. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

The storage device 730 is capable of providing mass storage for the computing device 700. In some implementations, the storage device 730 is a computer-readable medium. In various different implementations, the storage device 730 may be a floppy disk device, a hard disk device, an optical disk device, or a tape device, a flash memory or other similar solid state memory device, or an array of devices, including devices in a storage area network or other configurations. In additional implementations, a computer program product is tangibly embodied in an information carrier. The computer program product contains instructions that, when executed, perform one or more methods, such as those described above. The information carrier is a computer- or machine-readable medium, such as the memory 720, the storage device 730, or memory on processor 710.

The high speed controller 740 manages bandwidth-intensive operations for the computing device 700, while the low speed controller 760 manages lower bandwidth-intensive operations. Such allocation of duties is exemplary only. In some implementations, the high-speed controller 740 is coupled to the memory 720, the display 780 (e.g., through a graphics processor or accelerator), and to the high-speed expansion ports 750, which may accept various expansion cards (not shown). In some implementations, the low-speed controller 760 is coupled to the storage device 730 and a low-speed expansion port 790. The low-speed expansion port 790, which may include various communication ports (e.g., USB, Bluetooth, Ethernet, wireless Ethernet), may be coupled to one or more input/output devices, such as a keyboard, a pointing device, a scanner, or a networking device such as a switch or router, e.g., through a network adapter.

The computing device 700 may be implemented in a number of different forms, as shown in the figure. For example, it may be implemented as a standard server 700a or multiple times in a group of such servers 700a, as a laptop computer 700b, as part of a rack server system 700c, as a mobile device 700d (such as a smart phone), or as a tablet computer 700e.

Various implementations of the systems and techniques described herein can be realized in digital electronic and/or optical circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

A software application (i.e., a software resource) may refer to computer software that causes a computing device to perform a task. In some examples, a software application may be referred to as an "application," an "app," or a "program." Example applications include, but are not limited to, system diagnostic applications, system management applications, system maintenance applications, word processing applications, spreadsheet applications, messaging applications, media streaming applications, social networking applications, and gaming applications.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, non-transitory computer readable medium, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

The non-transitory memory may be physical devices used to store programs (e.g., sequences of instructions) or data (e.g., program state information) on a temporary or permanent basis for use by a computing device. The non-transitory memory may be volatile and/or non-volatile addressable semiconductor memory. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

The processes and logic flows described in this specification can be performed by one or more programmable processors, also referred to as data processing hardware, executing one or more computer programs to perform functions by operating on input data and generating output. The processes and logic flows can also be performed by special purpose logic circuitry, e.g., an FPGA (field programmable gate array) or an ASIC (application specific integrated circuit). Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, one or more aspects of the disclosure can be implemented on a computer having a display device, e.g., a CRT (cathode ray tube), LCD (liquid crystal display) monitor, or touch screen for displaying information to the user and optionally a keyboard and a pointing device, e.g., a mouse or a trackball, by which the user can provide input to the computer. Other kinds of devices can be used to provide interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

A number of implementations have been described. Nevertheless, it will be understood that various modifications may be made without departing from the spirit and scope of the disclosure. Accordingly, other implementations are within the scope of the following claims.

## Claims

1. A method of generating user interfaces based on conditions associated with users, comprising:
providing, by one or more processors, a first user interface comprising a first plurality of user interface elements to present first digital therapeutic content to a user, the first user interface configured to address one or more of a plurality of conditions via interactions with the first user interface;
identifying, by the one or more processors, a condition to be addressed in the user from the plurality of conditions, using data associated with the user;
generating, by the one or more processors, a second user interface to include (i) at least one of the first plurality of user interface elements of the first user interface and (ii) a second plurality of user interface elements to present second digital therapeutic content to address the condition identified for the user; and
providing, by the one or more processors, the second user interface comprising the second plurality of user interface elements to present the second digital therapeutic content to the user to address the condition identified for the user.

2. The method of claim 1, further comprising:
receiving, by the one or more processors, input data identifying the interactions by the user with the second plurality of user interface elements of the second user interface,
identifying, by the one or more processors, a second condition different from the first condition to be addressed in the user based on the input data; and
generating, by the one or more processors, a third user interface to include (i) the second user interface and (ii) a third plurality of user interface elements to provide third digital therapeutic content to address the second condition identified for the user.

3. The method of claim 1 or claim 2, wherein identifying the condition further comprises identifying, from the plurality of conditions, at least a first condition and a second condition to be addressed in the user, and
wherein generating the second user interface further comprises generating the second user interface to include (i) the second plurality of user interface elements for provision of the second digital therapeutic content to address the first condition and (ii) a third plurality of user interface elements for provision of third digital therapeutic content to address the second condition.

4. The method of any preceding claim, further comprising receiving, by the one or more processors, input data identifying the interactions by the user with the first plurality of user interface elements of the first user interface,
wherein identifying the condition further comprises determining a change in state associated with the user based on the input data on the first user interface, and
wherein generating the second user interface further comprises generating the second user interface responsive to determining the change in state.

5. The method of any preceding claim, further comprising selecting, by the one or more processors, from a plurality of user interface sets, a user interface set comprising at least a portion of the second plurality of user interface elements based on input data identifying the interactions by the user with the first plurality of user interface elements of the first user interface, and
wherein generating the second user interface further comprises generating the second user interface to include at least the portion of the second plurality of user interface elements defined by the user interface set; and preferably
wherein selecting the user interface set further comprises applying a machine learning model to the input data to select the user interface set from the plurality of user interface sets, wherein the machine learning model is establishing using a plurality of selections each identifying sample input data and a respective user interface set selected from the plurality of user interface sets.

6. The method of claim 1, further comprising:
receiving, by the one or more processors, input data identifying the interactions by the user with the second plurality of user interface elements of the second user interface, and
identifying, by the one or more processors, a change in state associated with the user based on the input data on the second user interface, and
determining, by the one or more processors, responsive to the change in state, to provide the first user interface comprising the first plurality of user interface elements to present the first digital therapeutic content to the user.

7. The method of any preceding claim, wherein identifying the condition further comprises receiving, from a remote computing system, an indication to provide a digital therapeutic associated with the second digital therapeutic content to the user to address the condition.

8. The method of any preceding claim, wherein the plurality of conditions comprises at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease; and/or
wherein the user is on a medication to address the condition, in partial concurrence with the presentation of at least one of the first digital therapeutic content or the second digital therapeutic content.

9. A system for generating user interfaces based on conditions associated with users, comprising:
one or more processors coupled with memory, configured to:
provide a first user interface a first user interface comprising a first plurality of user interface elements to present first digital therapeutic content to a user, the first user interface configured to address one or more of a plurality of conditions via interactions with the first plurality of user interface elements;
identify a condition to be addressed in the user from the plurality of conditions, using data associated with the user;
generate a second user interface to include (i) at least one of the first plurality of user interface elements of the first user interface and (ii) a second plurality of user interface elements to present second digital therapeutic content to address the condition identified for the user; and
provide the second user interface comprising the second plurality of user interface elements to present the second digital therapeutic content to the user to address the condition identified for the user.

10. The system of claim 9, wherein the one or more processors are configured to:
receive input data identifying the interactions by the user with the second plurality of user interface elements of the second user interface,
identify a second condition different from the first condition to be addressed in the user based on the input data; and
generate a third user interface to include (i) the second user interface and (ii) a third plurality of user interface elements to provide third digital therapeutic content to address the second condition identified for the user.

11. The system of claim 9 or claim 10, wherein the one or more processors are configured to:
identify, from the plurality of conditions, at least a first condition and a second condition to be addressed in the user;
generate the second user interface to include (i) the second plurality of user interface elements for provision of the second digital therapeutic content to address the first condition and (ii) a third plurality of user interface elements for provision of third digital therapeutic content to address the second condition.

12. The system of any one of claims 9 to 11, wherein the one or more processors are configured to:
receive input data identifying the interactions by the user with the first plurality of user interface elements of the first user interface,
determine a change in state associated with the user based on the input data on the first user interface; and
generate the second user interface responsive to determining the change in state; and preferably
select, from a plurality of user interface sets, a user interface set comprising at least a portion of the second plurality of user interface elements based on input data identifying the interactions by the user with the first plurality of user interface elements of the first user interface; and
generate the second user interface to include at least the portion of the second plurality of user interface elements defined by the user interface set; and preferably
wherein the one or more processors are configured to apply a machine learning model to the input data to select the user interface set from the plurality of user interface sets, wherein the machine learning model is establishing using a plurality of selections each identifying sample input data and a respective user interface set selected from the plurality of user interface sets.

13. The system of any one of claims 9 to 12, wherein the one or more processors are configured to:
receive input data identifying the interactions by the user with the second plurality of user interface elements of the second user interface;
identify a change in state associated with the user based on the input data on the second user interface; and
determine, responsive to the change in state, to provide the first user interface comprising the first plurality of user interface elements to present the first digital therapeutic content to the user.

14. The system of any one of claims 9 to 13, wherein the one or more processors are configured to receive, from a remote computing system, an indication to provide a digital therapeutic associated with the second digital therapeutic content to the user to address the condition.

15. The system of any one of claims 9 to 14, wherein the plurality of conditions comprises at least one of: substance use disorder, opioid use disorder, chronic insomnia, alcohol use disorder, schizophrenia, generalized anxiety disorder, major depressive disorder, bipolar, posttraumatic stress disorder, acute and chronic pain, migraine, multiple sclerosis, epilepsy, irritable bowel syndrome, specialty gastroenterology, cancer, or cardiovascular disease; and/or
wherein the user is on a medication to address the condition, in partial concurrence with the presentation of at least one of the first digital therapeutic content or the second digital therapeutic content.
